# EUROPEAN PATENT APPLICATION

(11) **EP 1 512 687 A1**
(43) Date of publication of application: **09.03.2005**
(21) Application number: 03020177.6
(22) Date of filing: 05.09.2003
(51) Int. Cl.: C07D 487/04, A61K 31/495, A61P 15/00

(54) **Piperazines as oxytocin agonists**

(71) Applicant: Ferring B.V., 2132 JX Hoofddorp (NL)
(72) Inventor: Hudson, Peter, 2300 Copenhagen S. (DK); Pitt, Gary Robert William, Salisbury Wiltshire, SP4 OAR (GB); Batt, Andrej Roman, Southampton SO17 2LJ (GB); Roe, Michael Bryan, Southampton, SO14 6RB (GB)
(74) Representative: Poulsen, Niels Jakob

(57) **Abstract**

Disclosed are novel compounds according to general formula I, 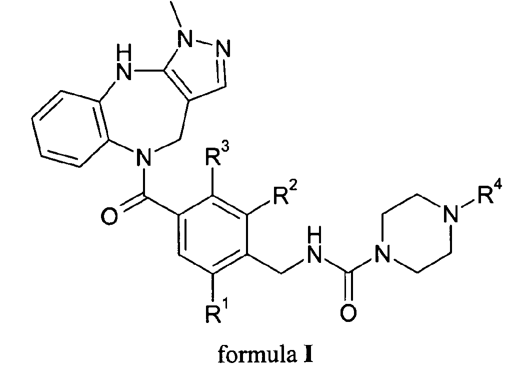 which have shown OT agonist activity.

## Description

### FIELD OF THE INVENTION

The present invention relates to a series of non-peptide oxytocin agonists and to pharmaceutical compositions comprising such compounds. The compositions are useful for the treatment of certain physiological disorders, such as erectile dysfunction.

### BACKGROUND

### Neurophyseal hormones

The neurophyseal hormones oxytocin (OT) and vasopressin (VP) are cyclic nonapeptides secreted by the posterior pituitary gland. These are described further e.g. p. 1-2 in the co-pending patent application, with the title "Oxytocin Agonists and Vasopressin Antagonists", which was filed 14 February 2003 (EP application number 03003394.8). The application describes oxytocin and vasopressin agonists and antagonists, p. 2 - 4. The application claims a number of V1a antagonists and their uses, but fails to disclose the present compounds.

The international application WO 03/016316, filed 6 August 2002, claims a number of compounds relating to the compounds of the present application, but fails to specifically disclose the present compounds.

There remains a need for alternative OT receptor agonists. They would be of utility for treating diseases related to inadequate oxytocin-like activity. Such compounds may advantageously be easy-to-synthesize non-peptides. Preferably they would be selective for the OT receptor over the VP receptors.

### SUMMARY OF THE INVENTION

According to a first aspect, the present invention concerns the compounds according to claim 1, and acceptable salts thereof.

According to a second aspect, the present invention relates to novel OT agonists, and pharmaceutically acceptable salts thereof.

According to a third aspect, the present invention relates to pharmaceutical compositions comprising these novel compounds, which compositions are useful for the treatment of, *inter alia,* male erectile dysfunction.

According to a fourth aspect, the present invention relates to the use of these novel compounds for the manufacture of a pharmaceutical composition, especially for the treatment of conditions for which inadequate oxytocin-like activity is implicated in the pathology.

According to further aspects, the present invention relates to the use of the above mentioned compounds and compositions in therapy and to therapeutic methods wherein the above mentioned compounds and compositions are used.

### DETAILED DESCRIPTION OF THE INVENTION

In a first aspect, the present invention comprises novel compounds according to general formula I, and pharmaceutically acceptable salts thereof.

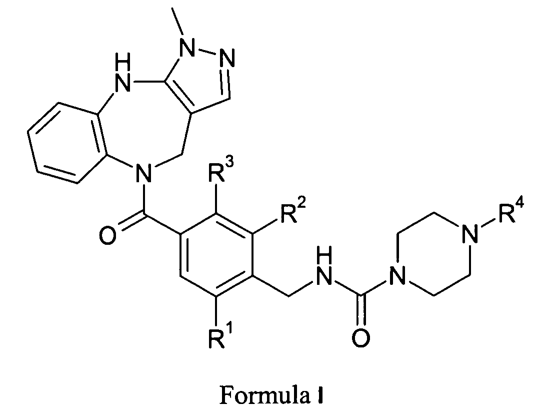

Here:
R1, R2 and R3 are each independently selected from H, alkyl, F and C1; and R4 is selected from formulae II, III and IV:

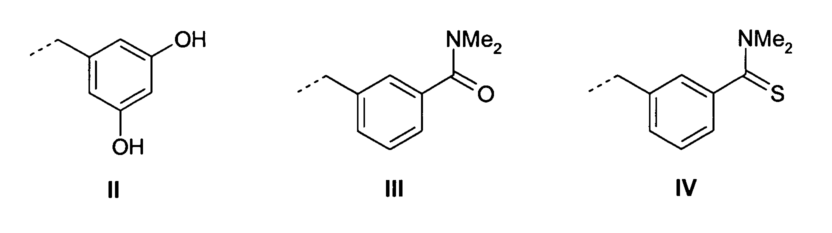

As used herein, the term "alkyl" is intended to designate lower alkyl groups, i.e. saturated hydrocarbon groups of between one and six carbon atoms, including linear, branched and cyclic alkyl groups. Examples of "alkyl" include, but are not limited to: C1 - methyl, C2 - ethyl, C3 - propyl, isopropyl, cyclopropyl, C4 - n-butyl, sec-butyl, isobutyl, tert-butyl, cyclobutyl, cyclopropylmethyl, methylcyclopropyl, C5 - n-pentyl, neopentyl, cyclopropylethyl, dimethylcyclopropyl, and C6 - n-hexyl, cyclohexyl, bicyclo[3.1.0]hexyl.

The compounds according to the present invention may have one or more stereogenic centres ("asymmetric carbon atoms") and so may exhibit optical isomerism. The scope of the present invention includes all epimers, enantiomers and diastereomers of compounds according to general formula I, including single isomers, mixtures and racemates.

The compounds according to the present invention generally contain a basic nitrogen atom and so are capable of forming addition salts with protic acids such as hydrochloric acid, sulphuric acid, phosphoric acid, acetic acid, trifluoroacetic acid, benzoic acid, maleic acid, citric acid, fumaric acid, methanesulphonic acid and the like.

Individual preferred compounds within the invention include:
4-(3,5-Dihydroxy-benzyl)-piperazine-1-carboxylic acid 2-methyl-4-(3-methyl-4,10-dihydro-3H-2,3,4,9-tetraaza-benzo[f] azulene-9-carbonyl)-benzylamide;
4-(3,5-Dihydroxy-benzyl)-piperazine-1-carboxylic acid 2,6-dimethyl-4-(3-methyl-4,10-dihydro-3H-2,3,4,9-tetraaza-benzo[f]azulene-9-carbonyl)-benzylamide;
4-(3,5-Dihydroxy-benzyl)-piperazine-1-carboxylic acid 3-chloro-4-(3-methyl-4,10-dihydro-3H-2,3,4,9-tetraaza-benzo[f]azulene-9-carbonyl)-benzylamide;
4-(3,5-Dihydroxy-benzyl)-piperazine-1-carboxylic acid 2-fluoro-4-(3-me thyl-4,10-dihydro-3H-2,3,4,9-tetraaza-benzo[f]azulene-9-carbonyl)-benzylamide;
4-(3-Dimethylcarbamoyl-benzyl)-piperazine-1 -carboxylic acid 2-methyl-4-(3-methyl-4,10-dihydro-3H-2,3,4,9-tetraaza-benzo[f]azulene-9-carbonyl)-benzylamide; and
4-(3-Dimethylthiocarbamoyl-benzyl)-piperazine-1-carboxylic acid 2-methyl-4-(3-methyl-4,10-dihydro-3H-2,3,4,9-tetraaza-benzo[f]azulene-9-carbonyl)-benzylamide.

These individually preferred compounds have several advantages over existing entities. These are specific OT agonists of high activity.

Furthermore, they are non-peptidic small molecules. It is well known that such compounds have significantly more potential to be orally active than peptides. As such they offer more convenience and better patient compliance than peptidic entities. Additionally, they are easily synthesized. Especially the individually preferred compounds comprise no chiral centers, thus allowing particularly simple synthesis.

The compounds of the present invention can be prepared by standard chemical manipulations. Such manipulations are e.g. described in EP application 03003394.8 p. 14,1. 32 - p. 20,1. 31 and WO 03/016316 p. 12 - 17.

The compounds according to the present invention are useful in human and/or animal therapy. When so used, they will generally be formulated in an appropriate manner. Thus a third aspect of the present invention is a pharmaceutical formulation that includes a compound as described above as an active ingredient. A fourth aspect of the present invention is the use of a compound according to the first aspect in the manufacture of such a composition.

The compounds according to the present invention are useful for treatment of several diseases, disorders or conditions. The term "treatment" used herein relates to both treatment in order to cure or alleviate a disease, disorder or a condition, and to treatment in order to prevent the development of a disease, disorder or a condition. The treatment may either be performed in an acute or in a chronic way. The human or animal to be treated, i.e. the patient, may be any human or non-human mammal in need of treatment according to the invention.

Further aspects of the invention relates to methods for treatment of the above mentioned diseases, disorders or conditions. According to the method according to the invention a therapeutically effective amount of the compound, or of the pharmaceutical composition, described above is administered to a patient in need of this treatment.

The term "therapeutically effective amount" relates to an amount that will lead to the desired therapeutical effect. The therapeutically effective amount will be determined by the attending physician taking into consideration all appropriate factors. Generally a single dose will comprise between 0.1 mg and 1000 mg, preferably between 1 mg and 250 mg, of the active compound according to the invention. The dose may be given on a single occasion or repeatedly. When given repeatedly, it may be given at regular intervals, such as once, twice or three times daily, or on demand, according to the condition being treated.

The pharmaceutical composition according to the present invention may be presented in any form that is known in the art. For example, the formulation may be presented as a tablet, capsule, powder, suppository, cream, solution or suspension, or in a more complex form such as an adhesive patch. The formulation will generally include one or more excipients, such as diluents, bulking agents, binding agents, dispersants, solvents, preservatives, flavouring agents and the like. Where the formulation is presented as a tablet or capsule the excipients may optionally include one or more agents to control the release of the active species, such as a coating of a polymer that is insoluble at low pH but soluble at neutral or high pH. Such a coating (known as an "enteric coating") prevents the release of the active agent in the stomach but allows its release in the intestines. The formulation may also include one or more additional pharmacologically active species. Preferably the formulation includes no such additional active agents.

In even further aspects, the present invention comprises the use of compositions according to the invention, and hence of the compounds of the invention, in human and/or animal therapy, and methods of treatment involving such use of the compositions and compounds. The novel compounds of the present invention are potent and selective OT agonists and so they and pharmaceutical compositions comprising them are useful in the treatment of conditions for which inadequate oxytocin-like activity is implicated in the pathophysiology. Such conditions include, but are not limited to: sexual disorders such as male erectile dysfunction, ejaculatory disorders and female sexual dysfunction, cancer of the prostate, breast, ovary and bones, osteoporosis, benign prostatic hyperplasia, post-partum bleeding, and depression. The compositions may also be used to induce labour or delivery of the placenta, to decrease arterial blood pressure, to decrease exaggerated responses to stress and to increase the nociceptive threshold.

In a preferred embodiment, the composition is used to treat male and/or female sexual dysfunction, and more preferably erectile dysfunction.

When used as therapeutic agents, the compositions of the present invention may be administered by any appropriate route that is known in the art. For example, they may be administered by the oral, buccal, sublingual, rectal, intravaginal, nasal, pulmonary or transdermal routes. Alternatively, they may be given by injection, including intravenous, subcutaneous and intramuscular injection.

For long-term treatment an alternative to repeated dosing may be the administration of a depot dose. For this method of administration the active agent is generally introduced into a matrix of biodegradable polymer, such as a copolymer of lactic and glycolic acids, and the formulation is given either subcutaneous (s.c.) or intramuscularly (i.m.) so as to form a deposit from which the active agent is released as the polymer degrades.

In order to decide whether or not a compound having general formula I is a selective OT agonist the compound may be assayed to determine its ability to stimulate the OT receptors in intact cells. In the assay, the compounds cause significant stimulation at concentrations of 30 µM or less. Preferred compounds cause significant stimulation at concentrations of 300 nM or less.

The present invention is further illustrated in the following examples, which are intended to demonstrate the application of the invention but not to limit the scope thereof.

### EXAMPLES

The following abbreviations have been used:
- Bu: butyl - alkyl residues may be further denoted as n (normal, i.e. unbranched), i (iso) and t (tertiary)
- DIEA: N,N-diisopropylethylamine
- DMF: dimethylformamide
- Et: ethyl
- EtOAc: ethyl acetate
- HOBt: 1-hydroxybenzotriazole
- HPLC: high pressure liquid chromatography
- h: hour(s)
- Me: methyl
- MS: mass spectrum
- NMR: nuclear magnetic resonance spectrum - NMR spectra were recorded in CDCl₃ unless otherwise indicated
- OVA: omithine vasotocin analogue
- pet. ether: petroleum ether boiling in the range 60-80°C
- Ph: phenyl
- Pn: pentyl
- Pr: propyl
- THF: tetrahydrofuran
- WSCD: water-soluble carbodiimide (N-ethyl-N'-(3-dimethylaminopropyl)carbodiimide hydrochloride

Examples A-G describe the synthesis of the intermediates. Compounds according to the present invention are described in Examples 1-6.

### Example A:

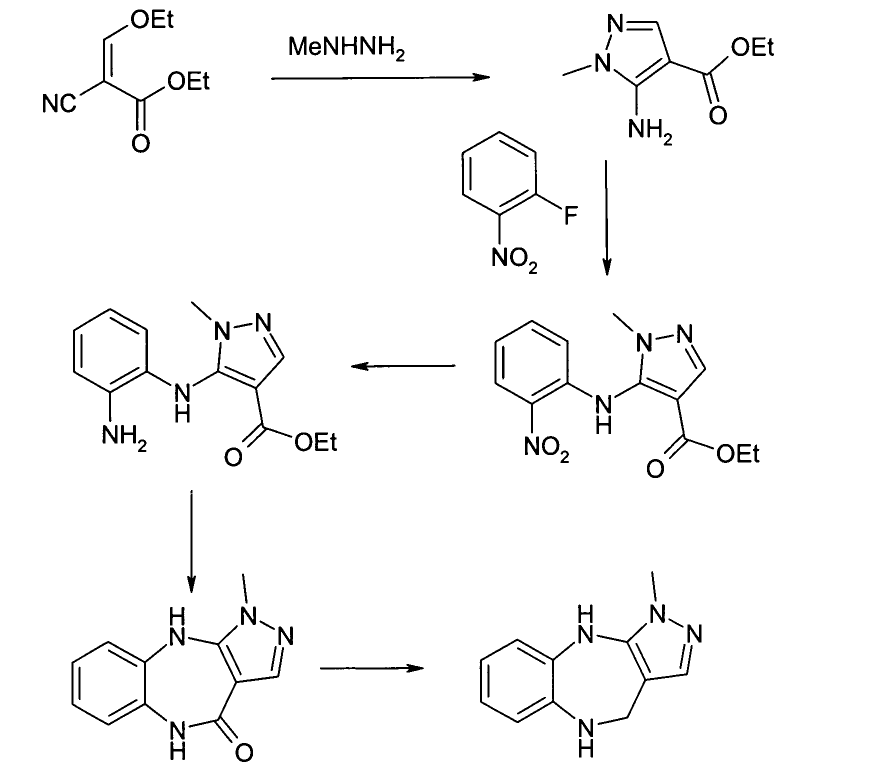

### 5-Amino-1-methyl-1H-pyrazole-4-carboxylic acid ethyl ester

Methylhydrazine (8.6ml, 162mmol) was added to a solution of ethyl (ethoxymethylene)cyanoacetate (24.9g, 147mmol) in ethanol (250ml) and the mixture was heated at reflux for 3 days. The solvent was *removed in vacuo* and the residue was purified by recrystallisation from EtOAc/pet. ether to yield a white solid identified as 5-amino-1-methyl-1*H*-pyrazole-4-carboxylic acid ethyl ester (21.1g,85%).

### 1-Methyl-5-(2-nitro-phenylamino)-1H-pyrazole-4-carboxylic acid ethyl ester

Sodium hydride (60% dispersion in oil, 7.0g, 170mmol) was added portionwise to a suspension of 5-amino-1-methyl-1*H* pyrazole-4-carboxylic acid ethyl ester (21.1g, 125mmol) in anhydrous THF (300ml) at 0°C. The mixture was allowed to warm to room temperature and stirred for 0.75h then cooled to 0°C. 1-fluoro-2-nitrobenzene (17.6g, 125mmol) was added and the resultant suspension was stirred at room temperature for 18h. EtOAc and 0.3M KHSO4 were added and separated. The aqueous layer was extracted with EtOAc and the combined organic layers were washed with brine, dried and concentrated in *vacuo.* The residue was purified by flash chromatography on silica gel (eluant 50% hexanes/50% ethyl acetate) to yield 1-methyl-5-(2-nitro-phenylamino)-1*H* pyrazole-4-carboxylic acid ethyl ester (20.8g, 58%).

### 5-(2-Amino-phenylamino)-1-methyl-1H pyrazole-4-carboxylic acid ethyl ester

1-Methyl-5-(2-nitro-phenylamino)-1*H* pyrazole-4-carboxylic acid ethyl ester (20.8g, 72mmol) was dissolved in methanol (330ml) and hydrogenated over 10% Pd/C catalyst for 4h. The mixture was filtered through Celite® filter agent and the filtrate was concentrated in vacuo to give a white solid identified as 5-(2-amino-phenylamino)-1-methyl-1*H* pyrazole-4-carboxylic acid ethyl ester (16.2g, 87%).

### 3-Methyl-4,9-dihydro-3H 2,3,4,9-tetraaza-benzo[f]azulen-10-one

A solution of 5-(2-amino-phenylamino)-1-methyl-1*H* pyrazole-4-carboxylic acid ethyl ester (3.38g, 13mmol) in acetic acid/2-propanol (1:9, 50ml) was heated at reflux for 4 days. The solvent was removed *in vacuo* and the residue was azeotroped with toluene and purified by recrystallisation from ethanol to yield 3-methyl-4,9-dihydro-3*H*-2,3,4,9-tetraaza-benzo[f]azulen-10-one (1.78g, 64%).

### 3-Methyl-3,4,9,10-tetrahydro-2,3,4,9-tetraaza-benzo[f]azulene

LiAlH₄ (1.21g, 33mmol) was added portionwise to a suspension of 3-methyl-4,9-dihydro-3*H*-2,3,4,9-tetraaza-benzo[f]azulen-10-one (1.78g, 8.3mmol) in anhydrous THF (25ml) at 0°C. The resulting suspension was heated at reflux for 18h, then allowed to cool to room temperature. A further portion of LiAlH₄ (600mg, 17mmol) was added and the mixture was heated at reflux for 6h. The mixture was cooled to 0°C, 35% ammonia solution (4ml) was added dropwise and the mixture was stirred for 0.5h. The resulting suspension was filtered through Celite® filter agent and the filtrate was concentrated *in vacuo* and azeotroped with toluene to give a solid identified as 3-methyl-3,4,9,10-tetrahydro-2,3,4,9-tetraaza-benzo[f]azulene (1.6g, 96%).

### Example B:

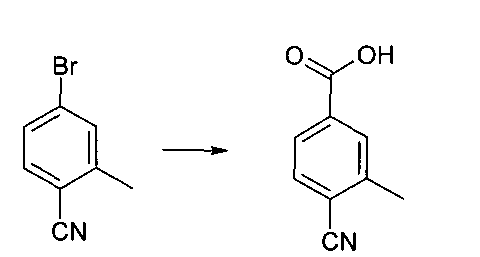

### 4-Cyano-3-methylbenzoic acid

To a solution of 4-bromo-2-methylbenzonitrile (2.0g, 10.2mmol) in THF (100ml) at -78°C under a nitrogen atmosphere was added dropwise a 2.5M solution of n-butyl lithium (4.48ml, 11.2mmol). The mixture was stirred at -78°C for 1h and then poured onto solid carbon dioxide (5g) in THF (50ml). The mixture was allowed to warm to room temperature. Water was added (200ml) and the mixture was extracted with diethyl ether (3 times). The aqueous layer was acidified by addition of concentrated HCl and extracted with chloroform (3 times). The combined chloroform extracts were washed with water, dried over MgSO4, and concentrated *in vacuo* to give a white solid identified as 4-cyano-3-methylbenzoic acid (1.2g, 73%).

### Example C:

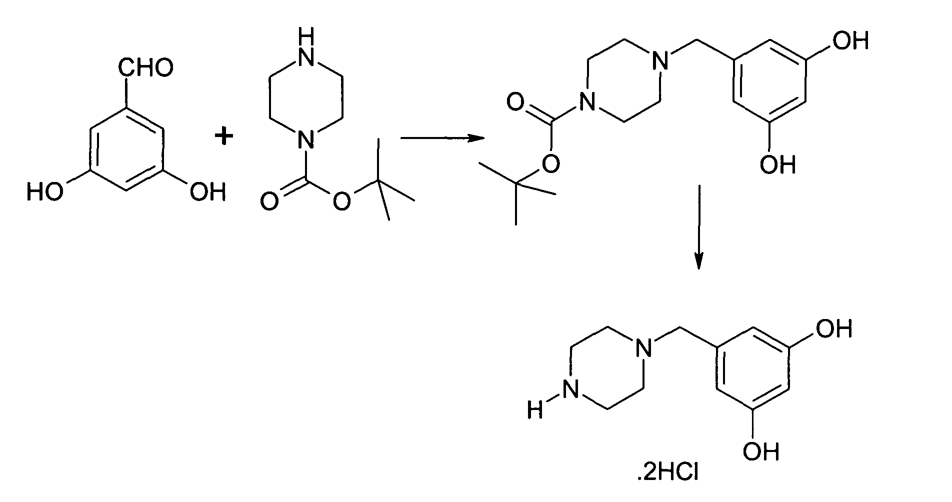

### 4-(3,5-Dihydroxy-benzyl)-piperazine-1-carboxylic acid tert-butyl ester

A solution of piperazine-1-carboxylic acid tert-butyl ester (1.56g, 8.4mmol) and 3,5-dihydroxybenzaldehyde (1.16g, 8.4mmol) in a mixture of methanol and acetic acid (99:1, 25ml) was stirred for 1h. Sodium cyanoborohydride (690mg, 10.9mmol) was added portionwise and the mixture was stirred for 3 days. The mixture was concentrated *in vacuo* and the residue was taken up in EtOAc and 5% KHCO₃. The mixture was separated and the organic layer was washed with brine, dried and reduced *in vacuo.* The residue was purified by flash column chromatography on silica get (eluant EtOAc:cyclohexane, 4:1) to afford a white foam identified as 4-(3,5-dihydroxy-benzyl)-piperazine-1-carboxylic acid tert-butyl ester (2.21g, 85%).

### 5-Piperazio-1-ylmethyl-benzene-1,3-diol dihydrochloride

A solution of 4M hydrogen chloride in dioxan (10ml) was added to a solution of 4-(3,5-dihydroxy-benzyl)-piperazine-1-carboxylic acid tert-butyl ester (2.21g, 7.2mmol) in methanol and the mixture was stirred for 1h. The mixture was *reduced in vacuo* and azeotroped with toluene to afford a white solid identified as 5-piperazin-1-ylmethyl-benzene-1,3-diol dihydrochloride (1.88g, 93%).

### Example D:

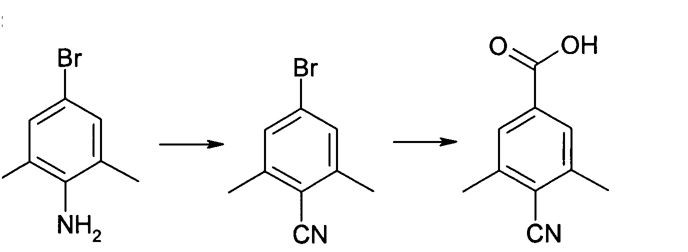

### 4-Bromo-2,6-dimethylbenzonitrile

4-Bromo-2,6-dimethylaniline (4.49g, 22.4mmol) was taken up in water (25ml) and concentrated hydrochloric acid (8.0ml) was added. The mixture was sonicated to form a fine suspension and then cooled to 0°C. A solution of sodium nitrite (1.67g, 24.2mmol) in water (5ml) was then added dropwise so as to maintain the temperature of the reaction between 0-5°C. The mixture was stirred at 0-5°C for 30 minutes and then neutralised by addition of solid sodium bicarbonate. The resulting solution was then added portionwise to a solution of copper cyanide (2.42g, 27.0mmol) and potassium cyanide (3.65g, 56.1mmol) in water (25ml) at 70°C. The mixture was stirred at 70°C for 30 minutes, allowed to cool and then extracted with toluene (2 times). The combined extracts were washed with water and brine, dried over MgSO4, and concentrated in vacuo. The residue was purified by flash chromatography on silica (eluant 5% ethyl acetate/ 95% pet. ether) to give an orange solid identified as 4-bromo-2,6-dimethylbenzonitrile (3.2g, 68%).

### 4-Cyano-3,5-dimethylbenzoic acid

4-Bromo-2,6-dimethylbenzonitrile (3.20g, 15.2mmol) was reacted following the method of Example B to give a tan solid identified as 4-cyano-3,5-dimethylbenzoic acid (1.5g, 56%).

### Example E:

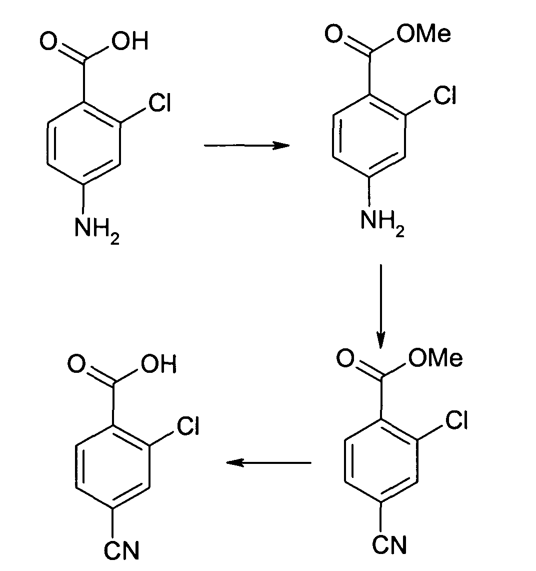

### 4-Amino-2-chlorobenzoic acid methyl ester

Acetyl chloride (2.5ml) was added drop-wise to a stirred solution of 4-amino-2-chlorobenzoic acid (2.22g, 12.9mmol) in methanol (75ml). The mixture was heated at reflux for 18h, cooled and reduced *in vacuo.* The residue was taken up in EtOAc, washed with saturated sodium hydrogen carbonate solution and brine, dried and reduced *in vacuo* to afford a beige solid identified as 4-amino-2-chlorobenzoic acid methyl ester, yield 2.32g, 97%.

### 2-Chloro-4-cyanobenzoic acid methyl ester

4-Amino-2-chlorobenzoic acid methyl ester (4.7g, 25mmol) was taken up in water (12ml) and concentrated hydrochloric acid (12ml) was added. The mixture was sonicated to form a fine suspension and then cooled to 0°C. A solution of sodium nitrite (1.91g, 27.7mmol) in water (15ml) was then added dropwise so as to maintain the temperature of the reaction between 0-5°C. The mixture was stirred at 0-5°C for 15 minutes and then neutralised by addition of solid sodium bicarbonate. The resulting solution was then added to a solution of copper cyanide (2.79g, 31mmol) and potassium cyanide (4.0g, 61mmol) in water (25ml) at 75°C. The mixture was stirred at 75°C for 45 minutes, allowed to cool and then extracted with toluene (2 times). The combined extracts were washed with brine, dried and concentrated *in vacuo.* The residue was purified by flash chromatography on silica (eluant 20% ethyl acetate/ 80% pet. ether) to give a pale orange solid identified as 2-chloro-4-cyanobenzoic acid methyl ester, yield 2.61g, 53%.

### 2-Chloro-4-cyanobenzoic acid

Lithium hydroxide monohydrate (1.18g, 28mmol) was added to a solution of 2-chloro-4-cyanobenzoic acid methyl ester (2.61g, 13mmol) in a mixture of THF and water (4:1, 100ml) and stirred for 18h. The mixture was reduced *in vacuo,* the residue was acidified with 1N hydrochloric acid and extracted with a mixture of methanol and chloroform (5:95). The organic extracts were washed with brine, dried and reduced. The residue was recrystallised from dioxan/pet. ether to give a pale orange solid identified as 2-chloro-4-cyanobenzoic acid, yield 1.34g, 55%.

### Example F

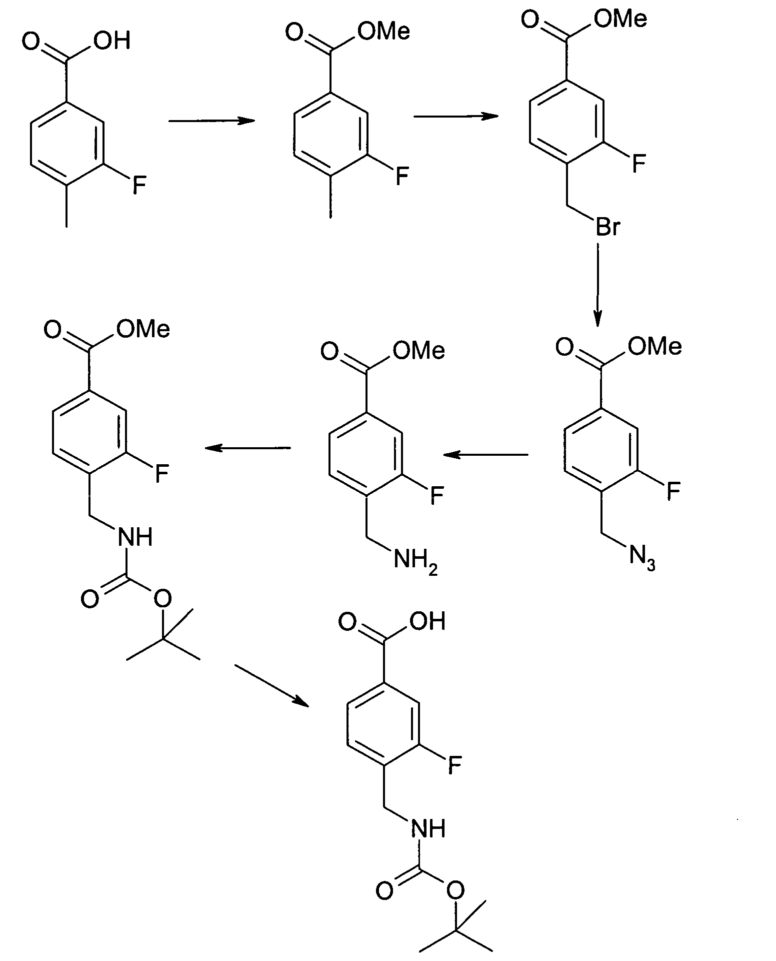

### 3-Fluoro-4-methylbenzoic acid methyl ester

Thionyl chloride was added to a solution of 3-fluoro-4-methylbenzoic acid (2.5g, 16.2mmol) in toluene (50ml) and heated at reflux for 3h. The mixture was cooled, reduced *in vacuo,* azeotroped with toluene and taken up in dichloromethane (30ml). The mixture was cooled in an ice/water bath and methanol (30ml) was added. The mixture was allowed to warm to room temperature, stirred for 3 days and reduced *in vacuo.* The residue was purified by flash column chromatography (eluant EtOAc:hexanes, 10:90) to afford a colourless oil identified as 3-fluoro-4-methylbenzoic acid methyl ester, yield 2.1g, 77%.

### 4-Bromomethyl-3-fluorobenzoic acid methyl ester

N-Bromosuccinimide (2.4g, 13.5mmol) and 2,2'-azobisisobutyronitrile (213mg, 1.3mmol) were added to a solution of 3-fluoro-4-methylbenzoic acid methyl ester (2.1g, 12.5mmol) in carbon tetrachloride (60ml) and heated at reflux for 18h. The mixture was cooled, filtered and reduced *in vacuo.* The residue was purified by flash column chromatography on silica gel (eluant EtOAc:hexanes, 10:90) to afford a colourless oil identified as 4-bromomethyl-3-fluorobenzoic acid methyl ester, yield 2.1g, 68%.

### 4-Azidomethyl-3-fluorobenzoic acid methyl ester

Sodium azide (609mg, 9.36mmol) was added to a solution of 4-bromomethyl-3-fluorobenzoic acid methyl ester (2.1g, 8.5mmol) in DMF (30ml) and stirred for 18h. The mixture was diluted with EtOAc, washed with water and brine, dried and reduced *in vacuo* to afford a colourless oil identified as 4-azidomethyl-3-fluorobenzoic acid methyl ester, yield 1.78g, 100%.

### 4-Aminomethyl-3-fluorobenzoic acid methyl ester

4-Azidomethyl-3-fluorobenzoic acid methyl ester (1.78g, 8.5mmol) was dissolved in methanol (50ml) and hydrogenated over 10% Pd/C catalyst (850mg) for 1h. The mixture was filtered through Celite® filter agent and the filtrate was concentrated in vacuo to give a solid identified as 4-aminomethyl-3-fluorobenzoic acid methyl ester, yield 1.53g, 98%.

### 4-(tert-Butoxycarbonylamino-methyl)-3-fluorobenzoic acid methyl ester

Di-tert-butyl-dicarbonate (2.19g, 10mmol) was added to a solution of 4-aminomethyl-3-fluorobenzoic acid methyl ester (1.53g, 8.5mmol) and triethylamine (1.75ml, 12.6mmol) in dichloromethane (30ml) and stirred for 18h. The mixture was reduced *in vacuo* and the residue was purified by flash column chromatography (eluant EtOAc:hexanes, 30:70) to afford a white solid identified as 4-(tert-butoxycarbonylamino-methyl)-3-fluorobenzoic acid methyl ester, yield 1.8g, 76%).

### 4-(tert-Butoxycarbonylamino-methyl)-3-fluorobenzoic acid

A solution of 1N sodium hydroxide (10ml) was added to a solution of 4-(tert-butoxycarbonylamino-methyl)-3-fluorobenzoic acid methyl ester (1.8g, 6.4mmol) in dioxan (25ml) and stirred for 1h. A further amount of sodium hydroxide solution was added and the mixture was stirred for 3 days. The mixture was reduced *in vacuo* and the residue was taken up in EtOAc, washed with 1N KHSO₄, water and brine, dried and reduced *in vacuo.* The residue was purified by flash column chromatography on silica gel (eluant hexanes:EtOAc:acetic acid, 90:9: 1) to afford a white solid identified as 4-(tert-butoxycarbonylaminomethyl)-3-fluorobenzoic acid, yield 1.08g, 64%.

### Example G

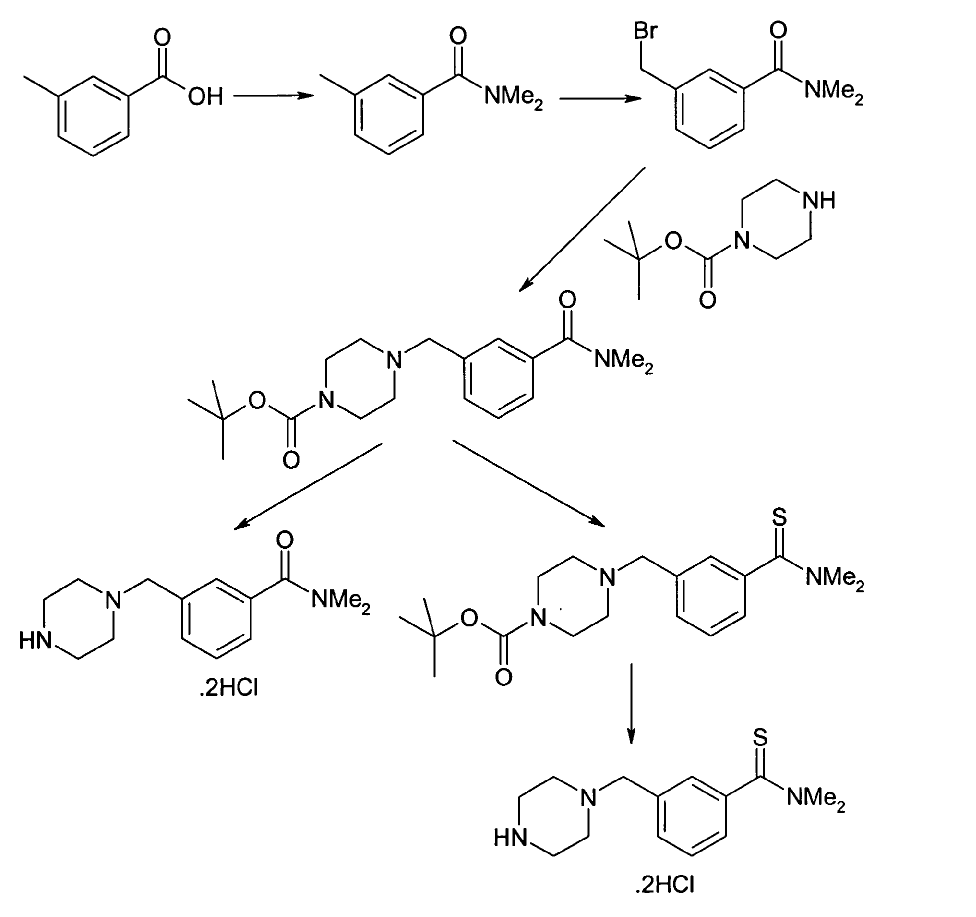

### 3,N,N Trimethylbenzamide

HOBt (5.96g, 44mmol) and WSCD (6.77g, 35.2mmol) were added to a solution of m-toluic acid (4.0g, 29.4mmol) in dichloromethane (40ml) while cooling in an ice/water bath. The mixture was allowed to warm to room temperature and stirred for 1h and then cooled in an ice/water bath. DIEA (15.4ml, 88.1mmol) and dimethylamine hydrochloride (3.59g, 44.1mmol) were added and the mixture was allowed to warm to room temperature. The mixture was stirred for 3 days, reduced *in vacuo* and the residue was partitioned between EtOAc and 2N HCl(aq). The organic layer was washed with 1N HCl(aq), water and brine, dried and reduced *in vacuo.* The residue was used without further purification.

### 3-Bromomethyl-N,N-dimethyl-benzamide

*N*-Bromosuccinimde (4.09g, 23mmol) and 2,2'-azobisisobutyronitrile (378mg, 2.3mmol) were added to a solution of crude *3,N,N* trimethylbenzamide from the above procedure in carbon tetrachloride (150ml) and heated at reflux for 18h. The mixture was cooled and *reduced in vacuo.* The residue was purified by flash column chromatography (eluant EtOAc:hexanes, 85:15) to afford a mixture of 3,*N,N*-trimethylbenzamide and 3-bromomethyl-*N,N*-dimethylbenzamide, yield 2.0g.

### 4-(3-Dimethylcarbamoyl-benzyl)-piperazine-1-carboxylic acid tert-butyl ester

The mixture of 3,*N,N-*trimethylbenzamide and 3-bromomethyl-*N,N*-dimethylbenzamide from the above procedure (2.0g) was added to a solution of piperazine-1-carboxylic acid tert-butyl ester (1.00g, 5.37mmol) and triethylamine (1.45ml, 10.3mmol) in dichloromethane (15ml) and stirred for 18h. The mixture was reduced *in vacuo* and the residue was purified by flash column chromatography (eluant EtOAc 100% then dichloromethane: methanol, 97.5:2.5) to afford 4-(3-dimethylcarbamoyl-benzyl)-piperazine-1-carboxylic acid tert-butyl ester, yield 655mg.

### N,N-Dimethyl-3-piperazin-1-ylmethyl-benzamide dihydrochloride

4-(3-Dimethylcarbamoyl-benzyl)-piperazine-1-carboxylic acid tert-butyl ester (322mg, 0.93mmol) was dissolved in a solution of 4M hydrogen chloride in dioxan (10ml) while cooling in an ice/water bath. The mixture was allowed to warm to room temperature and stirred for 1h. The mixture was reduced *in* *vacuo* to afford *N,N* dimethyl-3-piperazin-1-ylmethyl-benzamide dihydrochloride, yield 244mg, 82%.

### 4-(3-Dimethylthiocarbamoyl-benzyl)-piperazine-1-carboxylic acid tert-butyl ester

2,4-Bis(4-phenoxyphenyl)-1,3-dithia-2,4-diphosphetane-2,4-disulfide (279mg, 0.53mmol) was added to a solution of 4-(3-dimethylcarbamoyl-benzyl)-piperazine-1-carboxylic acid tert-butyl ester (333mg, 0.96mmol) in ethylene glycol dimethyl ether (10ml) and stirred for 18h. Flash column chromatography (eluant EtOAC:hexanes, 75:25) afforded 4-(3-dimethylthiocarbamoyl-benzyl)-piperazine-1-carboxylic acid tert-butyl ester, yield 110mg, 32%.

### N,N-Dimethyl-3-piperazin-1-ylmethyl-thiobenzamide dihydrochloride

4-(3-Dimethylthiocarbamoyl-benzyl)-piperazine-1-carboxylic acid tert-butyl ester (1 10mg, 0.30mmol) was dissolved in a solution of 4N hydrogen chloride in dioxan (8ml) while cooling in an ice/water bath. The mixture was allowed to warm to room temperature and stirred for 1h. The mixture was reduced in *vacuo* and azeotroped with toluene and diethyl ether to afford *N,N*-dimethyl-3-piperazin-1-ylmethyl-thiobenzamide dihydrochloride, yield 97mg, 96%.

### Example 1:

### 4-(3,5-Dihydroxy-benzyl)-piperazine-1-carboxylic acid 2-methyl-4-(3-methyl-4,10-dihydro-3H-2,3,4,9-tetraaza-benzo[f]azulene-9-carbonyl)-benzylamide

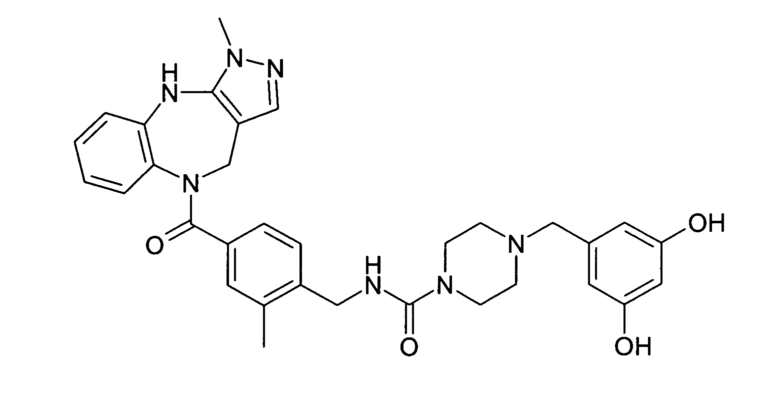

### 1A: 2-Methyl-4-(3-methyl-4,10-dihydro-3H-2,3,4,9-tetraazabenzo[f]azulene-9-carbonyl)-benzonitrile

Thionyl chloride (1.8ml, 27mmol) was added to a stirred suspension of 4-cyano-3-methylbenzoic acid from Example B (1.29g, 8.0mmol) in toluene (25ml). The mixture was heated at reflux for 2hr, cooled to room temperature and concentrated in vacuo. The residue was azeotroped with toluene then dissolved in dichloromethane (10ml). The resulting solution was added to a stirred suspension of 3-methyl-3,4,9,10-tetrahydro-2,3,4,9-tetraaza-benzo[f]azulene from Example A (1.6g, 8mmol) and triethylamine (1.4ml, 10mmol) in dichloromethane (15ml). The mixture was stirred overnight at room temperature then concentrated *in vacuo.* The residue was partitioned between chloroform and 0.3M KHSO₄. The aqueous phase was extracted with chloroform/2-propanol (80:20). The combined organic phases were washed with sat. Na-HCO₃ and brine, dried over Na₂SO₄ and concentrated *in vacuo.* The residue was purified by flash chromatography on silica gel (eluant 5% methanol/chloroform) to give a pale yellow solid identified as 2-methyl-4-(3-methyl-4,10-dihydro-3*H*-2,3,4,9-tetraazabenzo[f]azulene-9-carbonyl)-benzonitrile (2.4g, 87%).

### 1B: (4-Aminomethyl-3-methyl-phenyl)-(3-methyl-4,10-dihydro-3H-2,3,4,9-tetraaza-benzo[f]azulen-9-yl)-methanone

Cobalt(II) chloride hexahydrate (1.59g, 6.7mmol) was added to an ice-cold solution of 2-methyl-4-(3-methyl-4,10-dihydro-3H 2,3,4,9-tetraazabenzo[f]azulene-9-carbonyl)-benzonitrile (1.15g, 3.35mmol) in methanol (35ml). Sodium borohydride (1.27g, 33.5mmol) was added portionwise at 0°C and the mixture was stirred at room temperature for 1h, then quenched with 1M KHSO₄ and concentrated *in vacuo.* The aqueous residue was diluted with 1M KHSO₄ (40ml) and filtered through Celite® filter agent. The filtrate was washed with diethyl ether (2 x 50ml) then basified with 2M NaOH and extracted with chloroform. The organic phase was dried over Na₂SO₄ and concentrated *in vacuo* to give a pale brown solid identified as (4-aminomethyl-3-methyl-phenyl)-(3-methyl-4,10-dihydro-3H-2,3,4,9-tetraaza-benzo[f]azulen-9-yl)-methanone (745mg, 64%).

### 1C: 4-(3,5-Dihydroxy-benzyl)-piperazine-1-carboxylic acid 2-methyl-4-(3-methyl-4,10-dihydro-3H-2,3,4,9-tetraaza-benzo[f]azulene-9-carbonyl)-benzylamide

1,1'-Carbonyldiimidazole (128mg, 0.79mmol) and DIEA (250µl, 1.44mmol) were added to a solution of (4-aminomethyl-3 -methyl-phenyl)-(3 -methyl-4, 10-dihydro-3H-2,3,4,9-tetraaza-benzo[f]azulen-9-yl)-methanone (250mg, 0.72mmol) in DMF (5ml) and stirred for 1h. 5-Piperazin-1-ylmethyl-benzene-1,3-diol dihydrochloride from Example C (228mg, 0.81mmol) and DIEA (250µl, 1.44mmol) were added and the mixture stirred for 18h. The mixture was *reduced in vacuo* and the residue was taken up in EtOAc and a minimum amount of methanol. The mixture was washed with brine, *reduced in vacuo* and taken up again in EtOAc and a minimum amount of methanol. The mixture was washed with NaHCO₃ and brine, dried and reduced in *vacuo.* The residue was purified twice by flash column chromatography on silica gel (eluant CH₂Cl₂:methanol:ammonia solution, 90:9:1) and crystallised from EtOAc and methanol to afford a white powder identified as 4-(3,5-dihydroxy-benzyl)-piperazine-1 -carboxylic acid 2-methyl-4-(3-methyl-4,10-dihydro-3H-2,3,4,9-tetraaza-benzo[f]azulene-9-carbonyl)-benzylamide, yield 118mg, 28%.

^{**1**}**H NMR:** d₆-DMSO δ 2.10 (3H, s), 2.20-2.36 (4H, m), 3.21-3.41 (6H, m), 3.77 (3H, s), 3.87 (1H, d, J=14.4Hz), 4.09 (2H, s), 5.67 (1H, d, J=14.4Hz), 6.07 (1H, s), 6.15 (2H, s), 6.61-6.78 (2H, m), 6.85-6.93 (3H, m), 6.99 (1H, s), 7.06-7.20 (2H, m), 7.22-7.32 (1H, m), 8.62 (1H, s), 9.18 (2H, s) ppm.
**MS:** [M+H]⁺ = 582.3

### Example 4:

### 4-(3,5-Dihydroxy-benzyl)-piperazine-1-carboxylic acid 2-fluoro-4-(3-methyl-4,10-dihydro-3H-2,3,4,9-tetraaza-benzo [f]azulene-9-carbonyl)-benzylamide

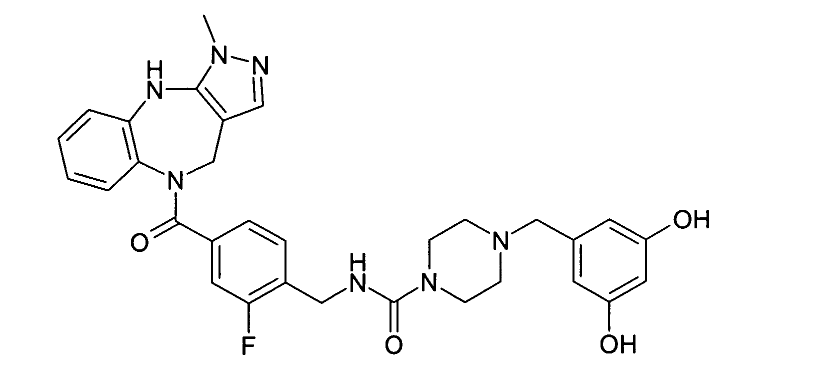

### 4A: [2-Fluoro-4-(3-methyl-4,10-dihydro-3H-2,3,4,9-tetraazabenzo[f]azulene-9-carbonyl)-benzyl]-carbamic acid tert-butyl ester

4-(tert-Butoxycarbonylamino-methyl)-3-fluorobenzoic acid from Example F (500mg, 1.86mmol) and an excess amount of triethylamine were dissolved in dichloromethane (20ml). WSCD (590mg, 3.08mmol) and 4-(dimethylamino)pyridine (200mg, 1.64mmol) were added and stirred for 0.5h. 3-Methyl-3,4,9,10-tetrahydro-2,3,4,9-tetraaza-benzo[f]azulene from Example A (309mg, 1.64mmol) was added and stirred for 48h. The mixture was reduced *in vacuo* and purified by flash column chromatography on silica gel (eluant EtOAc:hexanes, 50:50 to 100:0) to afford a pale tan foam identified as [2-fluoro-4-(3-methyl-4,10-dihydro-3H-2,3,4,9-tetraaza-benzo[f]azulene-9-carbonyl)-benzyl]-carbamic acid tert-butyl ester, yield 355mg, 51%.

### 4B: (4-Aminomethyl-3-fluoro-phenyl)-(3-methyl-4,10-dihydro-3H-2,3,4,9-tetraaza-benzo[f]azulen-9-yl)-methanone

[2-Fluoro-4-(3-methyl-4,10-dihydro-3H-2,3,4,9-tetraaza-benzo[f]azulene -9-carbonyl)-benzyl]-carbamic acid tert-butyl ester (335mg, 0.74mmol) was dissolved in a solution of 4M hydrogen chloride in dioxan (6ml) and stirred for 1h. The mixture was *reduced in vacuo* to afford a white solid identified as (4-aminomethyl-3-fluoro-phenyl)-(3-methyl-4,10-dihydro-3H-2,3,4,9-tetraaza-benzo[f]azulen-9-yl)-methanone, yield 288mg, 100%.

### 4C: 4-(3,5-Dihydroxy-benzyl)-piperazine-1-carboxylic acid 2-fluoro-4-(3-methyl-4,10-dihydro-3H-2,3,4,9-tetraaza-benzo[f]azulene-9-carbonyl)-benzylamide

(4-Aminomethyl-3-fluoro-phenyl)-(3-methyl-4,10-dihydro-3H-2,3,4,9-tetraaza-benzo[f]azulen-9-yl)-methanone (90mg, 0.23mmol) was reacted with 5-piperazin-1-ylmethyl-benzene-1,3-diol dihydrochloride from Example C (75mg, 0.27mmol) following the method of Example 1C to give a white solid identified as 4-(3,5-dihydroxy-benzyl)-piperazine-1-carboxylic acid 2-fluoro-4-(3-methyl-4,10-dihydro-3H-2,3,4,9-tetraaza-benzo[f]azulene-9-carbonyl)-benzylamide, yield 68mg, 50%.
^{**1**}**H NMR:** d₆-DMSO δ 2.20-2.32 (4H, m), 3.16-3.30 (6H, m), 3.77 (3H, s), 3.92 (1H, d, J=14.8Hz), 4.15 (2H, s), 5.65 (1H, d, J=14.8Hz), 6.07 (1H, s), 6.16 (2H, s), 6.60-7.30 (9H, m), 8.64 (1H, s), 9.16 (2H, s) ppm.
**MS**: [M+H]⁺ = 586.2

Examples 2, 3, 5 and 6 were prepared using analogous procedures to those described for Examples 1 and 4 above.

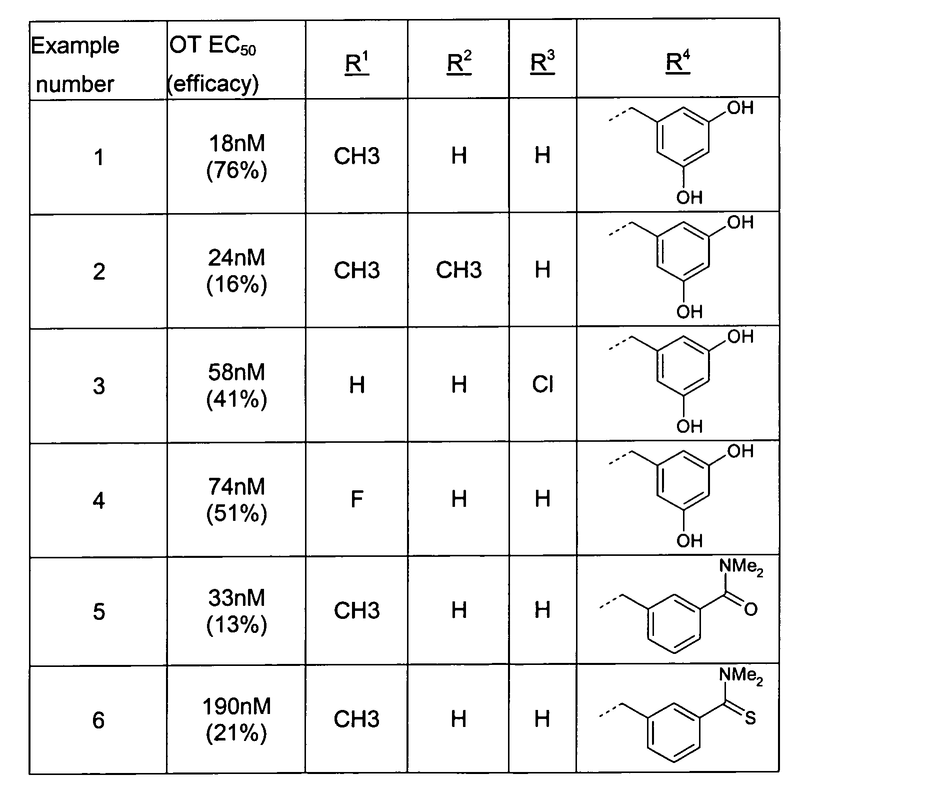

The names for these compounds are:

| Example number | Name |
|---|---|
| 1 | 4-(3,5-Dihydroxy-benzyl)-piperazine-1-carboxylic acid 2-methyl-4-(3-methyl-4,10-dihydro-3H-2,3,4,9-tetraaza-benzo[f]azulene-9-carbonyl)-benzylamide |
| 2 | 4-(3,5-Dihydroxy-benzyl)-piperazine-1-carboxylic acid 2,6-dimethyl-4-(3-methyl-4,1 0-dihydro-3H-2,3,4,9-tetraaza-benzo[f]azulene-9-carbonyl)-benzylamide |
| 3 | 4-(3,5-Dihydroxy-benzyl)-piperazine-1-carboxylic acid 3-chloro-4-(3-methyl-4,10-dihydro-3H-2,3,4,9-tetraaza-benzo[f]azulene-9-carbonyl)-benzylamide |
| 4 | 4-(3,5-Dihydroxy-benzyl)-piperazine-1-carboxylic acid 2-fluoro-4-(3-methyl-4,10-dihydro-3H-2,3,4,9-tetraaza-benzo[f]azulene-9-carbonyl)-benzylamide |
| 5 | 4-(3-Dimethylcarbamoyl-benzyl)-piperazine-1-carboxylic acid 2-methyl-4-(3-methyl-4,10-dihydro-3H-2,3,4,9-tetraaza-benzo[f]azulene-9-carbonyl)-benzylamide |
| 6 | 4-(3-Dimethylthiocarbamoyl-benzyl)-piperazine-1-carboxylic acid 2-methyl-4-(3-methyl-4,10-dihydro-3H-2,3,4,9-tetraaza-benzo[f]azulene-9-carbonyl)-benzylamide |

Mass spectra data for the exemplified compounds:

| Example number | MS: [M+ H]⁺ |
|---|---|
| 1 | 582.2 |
| 2 | 596.3 |
| 3 | 602.3, 604.1 (Cl³⁵, Cl³⁷) |
| 4 | 586.2 |
| 5 | 621.4 |
| 6 | 637.3 |

### Experimental Procedure

In order to decide whether or not examples 1-6 are agonists of the human OT receptor the compounds are applied to an assay in which a mammalian cell line e.g. Chinese hamster ovary (CHO) has been modified to express the human OT receptor either stably or transiently using standard transfection techniques. In addition, the cells are transfected with a gene for firefly luciferase under the control of a suitable promoter, e.g. NFAT promoter. Contact of the OT receptor by an agonist leads to stimulation of luciferase synthesis, the extent of which is measured using commercially available reagents. Concentrations at which the response is 50% of the maximum are calculated by fitting of data to 4-parameter logistic curves. Maximal efficacy is calculated compared to the maximal stimulation observed with OT.

All the cited references are hereby incorporated in their entirety.

## Claims

1. A compound according to general formula I, or a pharmaceutically acceptable salt thereof. 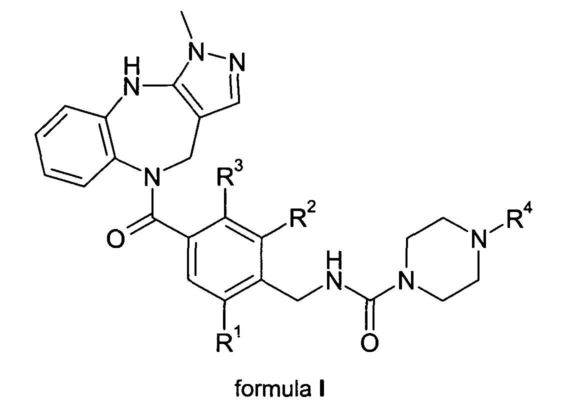 wherein:
R1, R2 and R3 are each independently selected from H, alkyl, F and Cl; and R4 is selected from formulae II, III and IV:

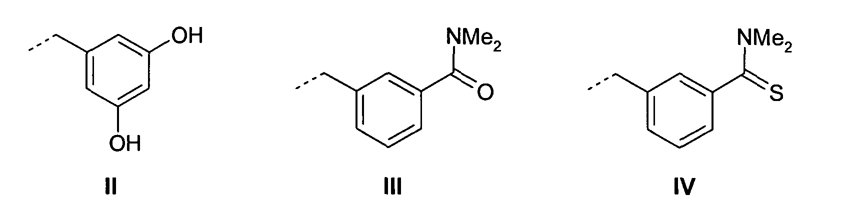

2. A compound according to claim 1, selected among the group consisting of:
4-(3,5-Dihydroxy-benzyl)-piperazine-1-carboxylic acid 2-methyl-4-(3-methyl-4,10-dihydro-3H-2,3,4,9-tetraaza-benzo [f] azulene-9-carbonyl)-benzylamide;
4-(3,5-Dihydroxy-benzyl)-piperazine-1-carboxylic acid 2,6-dimethyl-4-(3-methyl-4,10-dihydro-3H-2,3,4,9-tetraaza-benzo[f]azulene-9-carbonyl)-benzylamide;
4-(3,5-Dihydroxy-benzyl)-piperazine-1-carboxylic acid 3-chloro-4-(3-methyl-4,10-dihydro-3H-2,3,4,9-tetraaza-benzo[fJazulene-9-carbonyl)-benzylamide;
4-(3,5-Dihydroxy-benzyl)-piperazine-1-carboxylic acid 2-fluoro-4-(3-methyl-4,10-dihydro-3H-2,3,4,9-tetraaza-benzo[f]azulene-9-carbonyl)-benzylamide;
4-(3-Dimethylcarbamoyl-benzyl)-piperazine-1-carboxylic acid 2-methyl-4-(3-methyl-4,10-dihydro-3H-2,3,4,9-tetraaza-benzo[f]azulene-9-carbonyl)-benzylamide; and
4-(3-Dimethylthiocarbamoyl-benzyl)-piperazine-1-carboxylic acid 2-methyl-4-(3-methyl-4,10-dihydro-3H-2,3,4,9-tetraaza-benzo[f]azulene-9-carbonyl)-benzylamide.

3. A pharmaceutical composition comprising a compound according to claim 1 or 2 as an active agent.

4. A pharmaceutical composition according to claim 3, further comprising a pharmaceutical acceptable adjuvant, diluent or carrier.

5. A pharmaceutical composition according to claim 3 or 4, formulated as a tablet or capsule for oral administration.

6. A pharmaceutical composition according to any of the claims 3 - 5 for treatment of a disorder chosen among sexual disorders such as male erectile dysfunction, ejaculatory disorders and female sexual dysfunction; cancer of the prostate, breast, ovary and bones; osteoporosis; benign prostatic hyperplasia; post-partum bleeding and depression.

7. A pharmaceutical composition according to claim 6 for treatment of male erectile dysfunction.

8. The use of a compound according to claim 1 or 2, or a pharmaceutically acceptable salt thereof, for the for treatment of a disorder chosen among sexual disorders such as male erectile dysfunction, ejaculatory disorders and female sexual dysfunction; cancer of the prostate, breast, ovary and bones; osteoporosis; benign prostatic hyperplasia; post-partum bleeding and depression.

9. The use of a compound according to claim 1 or 2, or a pharmaceutically acceptable salt thereof, to induce labour or delivery of the placenta, to decrease arterial blood pressure, to decrease exaggerated responses to stress or to increase the nociceptive threshold.
